(19) Europäisches Patentamt
European Patent Office
Office européen des brevets

(11) EP 3 230 464 B1

(12) **EUROPEAN PATENT SPECIFICATION**

(45) Date of publication and mention of the grant of the patent:
**19.09.2018 Bulletin 2018/38**

(21) Application number: **15805513.7**

(22) Date of filing: **08.12.2015**

(51) Int Cl.:
*C12P 19/04* (2006.01) *C08B 37/00* (2006.01)
*C12M 1/00* (2006.01)

(86) International application number:
**PCT/EP2015/079004**

(87) International publication number:
**WO 2016/091892 (16.06.2016 Gazette 2016/24)**

(54) **PROCESS FOR THE FERMENTATION OF FUNGAL STRAINS**

VERFAHREN ZUR VERGÄRUNG VON PILZSTÄMMEN

PROCÉDÉ POUR LA FERMENTATION DE SOUCHES FONGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2014 EP 14197750**

(43) Date of publication of application:
**18.10.2017 Bulletin 2017/42**

(73) Proprietor: **Wintershall Holding GmbH**
**34119 Kassel (DE)**

(72) Inventors:
- **BRIECHLE, Sebastian**
**67227 Frankenthal (DE)**
- **HOLLMANN, Rajan**
**49152 Bad Essen (DE)**
- **KAEPPLER, Tobias**
**67133 Maxdorf (DE)**
- **LEHR, Florian**
**67365 Schwegenheim (DE)**
- **SCHMIDT, Julia Kristiane**
**69115 Heidelberg (DE)**
- **FREYER, Stephan**
**67434 Neustadt (DE)**

(74) Representative: **BASF IP Association**
**BASF SE**
**G-FLP-C006**
**67056 Ludwigshafen (DE)**

(56) References cited:
**EP-A1- 0 504 673** **EP-A2- 0 271 907**
**WO-A2-03/016545** **DE-A1- 4 012 238**

- **JINLI ZHANG ET AL: "High shear mixers: A review of typical applications and studies on power draw, flow pattern, energy dissipation and transfer properties", CHEMICAL ENGINEERING AND PROCESSING, ELSEVIER SEQUOIA, LAUSANNE, CH, vol. 57, 24 April 2012 (2012-04-24), pages 25-41, XP028510612, ISSN: 0255-2701, DOI: 10.1016/J.CEP.2012.04.004 [retrieved on 2012-05-01]**
- **A Haarstrick ET AL: "Cross-flow filtration as a method of separating fungal cells and purifying the polysaccharide produced", Bioprocess Engineering, 1 April 1991 (1991-04-01), pages 179-186, XP055196106, DOI: 10.1007/BF00369256 Retrieved from the Internet: URL:http://link.springer.com/content/pdf/10.1007/BF00369256.pdf [retrieved on 2015-06-15] cited in the application**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### Field of the invention

The present invention relates to a process for the fermentation of fungal strains which secrete glucans with a β-1,3-glycosidically linked main chain and side groups β-1,6-glycosidically bonded thereto, in a cascade of tanks using high-shear mixers.

### Background of the invention

In natural occurrences of petroleum, petroleum is present in the voids of porous storage rocks, which are closed to the Earth's surface by impermeable covering layers. The voids may be very fine voids, capillaries, pores or the like. Fine pore necks can have, for example, a diameter of only about 1 m. Apart from petroleum, including proportions of natural gas, a reservoir comprises more or less salt-comprising water.

In petroleum recovery, a distinction is made between primary, secondary and tertiary recovery. In primary recovery, the petroleum flows spontaneously under the reservoir's own pressure through the well to the surface after drilling down to the reservoir. Depending on the reservoir type, it is usually only possible, however, to recover about 5 to 10% of the amount of petroleum present in the reservoir by means of primary recovery; then, the intrinsic pressure is no longer sufficient for recovery. In secondary recovery, the pressure in the reservoir is maintained by injection of water and/or steam, but the petroleum cannot be fully recovered even with this technology. Tertiary petroleum recovery includes processes in which suitable chemicals are used as auxiliaries for oil recovery. These include so-called "polymer flooding". In polymer flooding, an aqueous solution of a thickener polymer is injected into the petroleum reservoir via the injection wells instead of water. This enables the yield to be increased further compared with the use of water or steam.

Suitable thickening polymers for tertiary petroleum recovery (also known as enhanced oil recovery (EOR)) must meet a number of specific requirements. In addition to sufficient viscosity, the polymers must also be thermally very stable and retain their thickening effect even at high salt concentrations.

A large number of different water-soluble polymers have been proposed for polymer flooding, specifically both synthetic polymers, such as polyacrylamides or copolymers comprising acrylamide and other monomers, and also water-soluble polymers of natural origin.

An important class of polymers of natural origin for polymer flooding is formed by branched homopolysaccharides from glucose. Polysaccharides composed of glucose units are also called glucans. The specified branched homopolysaccharides have a main chain composed of -1,3-linked glucose units, of which, statistically, about each third unit is -1,6-glycosidically linked with a further glucose unit. Aqueous solutions of such branched homopolysaccharides have advantageous physicochemical properties, meaning that they are particularly well suited to polymer flooding.

Particularly important glucans in this context are beta-glucans. Beta-glucans are known well-conserved components of cell walls in several microorganisms, particularly in fungi and yeast (Novak, Endocrine, Metabol & Immune Disorders - Drug Targets (2009), 9: 67-75). Biochemically, beta-glucans are non-cellulosic polymers of beta-glucose linked via glycosidic beta(1-3) bonds exhibiting a certain branching pattern with beta(1-6) bound glucose molecules (Novak, loc cit). A large number of closely related beta-glucans exhibit a similar branching pattern such as schizophyllan, scleroglucan, pendulan, cinerian, laminarin, lentinan and pleuran, all of which exhibit a linear main chain of beta-D-(1-3)-glucopyranosyl units with a single beta-D-glucopyranosyl unit (1-6) linked to a beta-D-glucopyranosyl unit of the linear main chain with an average branching degree of about 0,3 (Novak, loc cit; EP-B1 463540; Stahmann, Appl Environ Microbiol (1992), 58: 3347-3354; Kim, Biotechnol Letters (2006), 28: 439-446; Nikitina, Food Technol Biotechnol (2007), 45: 230-237). At least two of said beta-glucans - schizophyllan and scleroglucan - even share an identical structure and differ only slightly in their molecular mass, i.e. in their chain length (Survase, Food Technol Biotechnol (2007), 107-118).

Homopolysaccharides of said structure are secreted by various fungal strains, for example by the filamentously growing basidiomycete Schizophyllum commune, which secretes, during growth, a homopolysaccharide of said structure having a typical molecular weight Mw of about 5 to about $25*10^6$ g/mol (trivial name schizophyllan). Mention is also to be made of homopolysaccharides of said structure secreted by Sclerotium rolfsii (trivial name: scleroglucans).

Processes for producing branched homopolysaccharides from -1,3-linked glucose units by fermentation of fungal strains are known.

EP 0 271 907 A2 and EP 0 504 673 A1 disclose processes and fungal strains for producing branched homopolysaccharides composed of -1,3-linked glucose units in the main chain. Production takes place by discontinuous fermentation of the strains with stirring and aeration. The nutrient medium consists essentially of glucose, yeast extract, potassium dihydrogen phosphate, magnesium sulfate and water. The polymer is secreted by the fungus into the aqueous fermentation broth, and ultimately an aqueous polymer solution is separated off from the biomass-comprising fermentation broth, for example by centrifugation or filtration.

DE 40 12 238 A1 discloses a process for increasing the space-time yield during the production of nonionic

biopolymers, in particular of the fungal strains disclosed in EP 0 271 907 A2. To increase the space-time yield, the limitation of oxygen on the one hand, and furthermore the shearing of the cell walls with homogenization of the fermentation broth, and also the avoidance of pellet formation during the cultivation is disclosed. For the shearing, a toothed-wheel pump in a bypass is proposed.

Processes for fermentation of fungal strains are known for example from EP 0 271 907 A2, EP 0 504 673 A1, DE 40 12 238 A1, WO 03/016545 A2.

In particular, EP 0 271 907 A2, EP 0 504 673 A1 and DE 40 12 238 A1 disclose processes for the preparation, i.e. the preparation is effected by batchwise fermentation of the fungus Schizophyllum commune with stirring and aeration. The culture medium substantially comprises glucose, yeast extract, potassium dihydrogen phosphate, magnesium sulfate and water. EP 0 271 907 A2 describes a method for separating the polysaccharide, in which the culture suspension is first centrifuged and the polysaccharide is precipitated from the supernatant with isopropanol. A second method comprises a pressure filtration followed by an ultrafiltration of the solution obtained, without details of the method having been disclosed. "Udo Rau, "Biosynthese, Produktion und Eigenschaften von extrazellulären Pilz-Glucanen", Habilitationsschrift, Technical University of Brunswick, 1997, pages 70 to 95" and "Udo Rau, Biopolymers, Editor A. Steinbüchel, Volume 6, pages 63 to 79, WILEY-VCH Publishers, New York, 2002" describe the preparation of schizophyllan by continuous or batchwise fermentation. To prevent pellet formation, the fermentation broth was circulated in an external circuit equipped with a toothed-wheel pump. "GIT Fachzeitung Labor 12/92, pages 1233 - 1238" describes a continuous preparation of branched beta-1,3-glucans with cell recycling. WO 03/016545 A2 discloses a continuous process for the preparation of scleroglucans using Sclerotium rolfsii.

US 5,010,186 and US 4,873,323 disclose polysaccharide biopolymers having improved filterability that are prepared by acidifying an aqueous polysaccharide composition with nitric acid to a pH value from about 2 to 0.1 and treating said acidified composition at a temperature from about 50 °C to 100 °C for about 5 to 60 minutes.

US 4,667,026 describes an aqueous solution of polysaccharide biopolymers that are heat treated for more than 5 minutes at a pH value ranging from 3.5 to 6.2 to improve the filterability thereof.

It is essential that an aqueous solution comprising at least one beta-glucan that is used for polymer flooding does not comprise any gel particles or other small particles at all. Even a small number of particles having dimensions in the micron range blocks the fine pores in the mineral oil-containing formation and thus at least complicates or even stops the mineral oil production.

Consequently, it is therefore also important that aqueous solutions containing at least one beta-glucan are substantially free of cells and cell fragments because these cells and/or cell fragments otherwise block the mineral oil formation which complicates the extraction of the mineral oil or even makes it impossible. The so-called Filtration Ratio (FR value) can be used for characterizing the quality of aqueous solutions comprising at least one beta-glucan.

In principle the removal of cell fragments, gel particles and other small particles could be improved by using filter membranes with a small pore size. With decreasing pore size, however, the filter membranes increasingly also retain the beta-glucans, in particular the fractions of the beta-glucans that have very high molecular weights. The retention of beta-glucans with a very high molecular weight leads to a loss of beta-glucans and makes the overall process for the production of beta-glucans less economical.

In addition, an aqueous solution of beta-glucans is susceptible to bacterial invasion. As the beta-glucan acts as a nutrient for the bacteria, the beta-glucans are degraded. Upon degradation of the beta-glucans undesirable products from the metabolism of the bacteria are excreted into the aqueous solution such as for example hydrogen sulfide. Due to the formation of unwanted products the aqueous solution containing at least one beta-glucan cannot be used for oil field applications. Overall, even though processes for producing branched homopolysaccharides form -1,3-linked glucose units by fermentation of fungal strains are known from the prior art, there is a problem with the production in that the fungi have a tendency towards pellet formation during growth. If the size of the pellets exceeds a diameter of about 0.3 cm, the fungi in the core of the pellets can no longer be supplied adequately with oxygen. The phenomenon of oxygen limitation arises. This condition then leads to cell death of the fungi and to the formation of undesired byproducts such as ethanol, meaning that the space-time yield during the production of the homopolysaccharides is drastically reduced.

The methods specified in the prior art for preventing the pellet formation of fungi are not adequately reproducible and can therefore not be used for processes which are carried out on an industrial scale, carried out in particular in fermentation tanks larger than 5 $m^3$. There was therefore the need to provide further processes for the fermentation of fungal strains which make it possible to produce homopolysaccharides with a high space-time yield (STY).

One object of the present invention is thus to provide a process for the fermentation of fungal strains which secrete glucans with a β-1,3-glycosidically linked main chain and side groups β-1,6-glycosidically bonded thereto, which makes it possible to produce glucans with a β-1,3-glycosidically linked main chain and side groups β-1,6-glycosidically bonded thereto with a high space-time yield.

Summary of the Invention

The present invention provides a process for the fermentation of fungal strains which secrete glucans with a β-1,3-glycosidically linked main chain and side groups β-1,6-glycosidically bonded thereto according to the subject matter of the independent claim(s). Further embodiments are incorporated in the dependent claims.

According to an exemplary embodiment there is provided a process for the fermentation of fungal strains which secrete glucans with a β-1,3-glycosidically linked main chain and side groups β-1,6-glycosidically bonded thereto, in a cascade of tanks comprising at least a first tank with a first volume and a second tank with a second volume, comprising at least the steps

a) fermentation of the fungal strains in a first aqueous medium in the first tank and the volume of the first aqueous medium, resulting in a first mixture,

b) transfer of the first mixture to the second tank, and

c) fermentation of the fungal strains in the first mixture in a second aqueous medium in the second tank and the volume of the second aqueous medium, resulting in a second mixture, where the proportion of the volume of the first mixture to the volume of the second tank is in the range between ≥ 0.1% to ≤ 50% and where the first mixture in step b) is passed through at least one shear mixer or high-shear mixer, the shear mixer or high-shear mixer has a shearing geometry, such that the entire first mixture entirely passes through the shearing geometry of the at least one shear mixer or high-shear mixer.

Thus, it is possible to treat the first mixture by a high shear mixing process in order to keep a size of fungal agglomerates small before a second fermentation step starts. This will significantly increase the efficiency of the fermentation process. The agglomerates grow in size during fermentation. Larger agglomerates have a lower relative fermentation rate, so that it may be desirable keeping the size of the agglomerates lower. By treating the agglomerates with a shearing procedure the size decreases, so that the later fermentation process is more efficient.

As the process for fermentation of glucans with a fermentation step in a first tank and a further fermentation in a second tank includes a transfer from the first tank to the second tank includes a high shear mixing, such that the entire mixture passes through the shearing geometry of the high shear mixer, the shearing result becomes more homogenous than a shearing in a batch process, where the high shear mixer is arranged within the tank. In the latter case some parts of the mixture regularly pass the high shear mixer twice or more, while other parts do not pass the high shear mixer at all. Consequently without a high shear mixing during which the entire mixture passes through the shearing geometry of the high shear mixer a broad spectrum of particle size occurs including very small (twice or more passed) and very large (not passed) particles. The invention however, provides a procedure in which a high shear mixer in a flow process urges the entire mixture to pass the mixer, so that the spectrum of particle size is much smaller, as each part of the mixture passes the high shear mixer geometry. A small spectrum results in a more equalized subsequent fermentation, so that the in line high shear mixer of the invention results in a better, faster and more reliable fermentation result. Therefore, batch mixers, like e.g. an Ultraturax machine for a shearing process, do not result in comparable fermentation results.

Glucans are a class of homopolysaccharides whose monomer building block is exclusively glucose. The glucose molecule can be α-glycosidically or β-glycosidically linked, branched to varying degrees or be linear. Preference is given to glucans selected from the group consisting of cellulose, amylose, dextran, glycogen, lichenin, laminarin from algae, pachyman from tree fungi and yeast glucans with β-1,3 bonding; nigeran, a mycodextran isolated from fungi (α-1,3-glucan, α-1,4-glucan), curdlan (β-1,3-D-glucan), pullulan (α-1,4-bonded and α-1,6-bonded D-glucan) and schizophyllan (β-1,3 main chain, β-1,6 side chain) and pustulan (β-1,6-glucan).

The glucan preferably comprises a main chain composed of β-1,3-glycosidically linked glucose units and side groups composed of glucose units and β-1,6-glycosidically bonded to the main chain. The side groups preferably consist of a single β-1,6-glycosidically bonded glucose unit, with, statistically, each third unit of the main chain being β-1,6-glycosidically bonded with a further glucose unit. Depending on the source and method of isolation, beta-glucans have various degrees of branching and linkages in the side chains.

Generally, in context with the presently claimed invention, the beta-glucan as described herein may be any beta-glucan such as beta-1,4-glucans, beta-1,3-glucans, beta-1,6-glucans and beta-1,3(1,6)-glucans. In one embodiment, the beta-glucan is a polymer consisting of a linear main chain of beta-D-(1-3)-glucopyranosyl units having a single beta-D-glucopyranosyl unit (1-6) linked to a beta-D-glucopyranosyl unit of the linear main chain with an average branching degree of about 0.3. In context with the presently claimed invention, the term "average branching degree about 0.3" means that in average about 3 of 10 beta-D-(1-3)-glucopyranosyl units are (1-6) linked to a single beta-D-glucopyranosyl unit. In this context, the term "about" means that the average branching degree may be within the range from 0.25 to 0.35, preferably from 0.25 to 0.33, more preferably from 0.27 to 0.33, most preferably from 0.3 to 0.33. It may also be 0.3 or 0.33. The average branching degree of a beta-glucan can be determined by methods known in the art, e.g., by periodic oxidation analysis, methylated sugar analysis and NMR (Brigand, Industrial Gums, Academic Press, New

York/USA (1993), 461-472).

In the context of the presently claimed invention, the at least one beta-glucan to be produced as described herein is preferably selected from the group consisting of schizophyllan and scleroglucan, particularly preferably the at least one beta-glucan is schizophyllan.

Schizophyllan and scleroglucan can both be referred to as beta-1,3-glucans. Schizophyllan and scleroglucan have an average branching degree between 0.25 and 0.33 (Novak, *loc cit*, Survase, *loc cit*); for example, scleroglucan and schizophyllan have an average branching degree of 0.3 to 0.33. The polysaccharide chains usually form a three-dimensional structure of triple helices; polymer chains consist of glucose units whose hydroxy groups in 1- and 3-position are beta-linked to form the polymer main chain, and wherein each third glucose unit contains in position 6 a further glucose moiety linked by its hydroxyl function in position 1 (beta-1,3-bonded glucopyranose as the main chain and beta-1,6-bonded glucopyranose as side chains) and has the structural formula, where n is a number in the range from 7000 to 35 000:

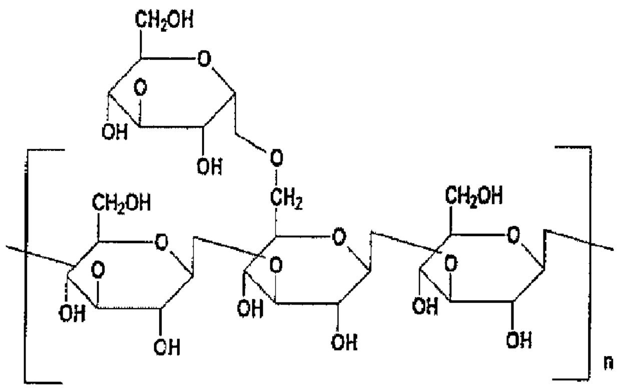

wherein n is a number which provides the beta-1,3-glucan component with a weight average molecular weight (Mw) of $5 \cdot 10^5$ g/mol to $25 \cdot 10^6$ g/mol, which is determined by GPC (Gel-Permeation-Chromatography) .

Fungal strains that secrete such glucans are known to the person skilled in the art. The fungal strains are preferably selected from the group consisting of Schizophyllum commune, Sclerotium rolfsii, Sclerotium glucanicum, Monilinia fructigena, Lentinula edodes and Botrytis cinera. Suitable fungal strains are also mentioned, for example, in EP 0 271 907 A2 and EP 0 504 673 A1. The fungal strains used are particularly preferably Schizophyllum commune or Sclerotium rolfsii and very particularly preferably Schizophyllum commune. This fungal strain secretes a glucan in which, on a main chain composed of β-1,3-glycosidically linked glucose units, each third unit - viewed statistically - of the main chain is β-1,6-glycosidically linked with a further glucose unit; i.e. the glucan is preferably the so-called schizophyllan.

Typical schizophyllans have a weight-average molecular weight Mw of about $5 \cdot 10^5$ g/mol to $25 \cdot 106$ g/mol.

The fungal strains are fermented in a suitable aqueous medium or nutrient medium. In the course of the fermentation, the fungi secrete the aforementioned class of glucans into the aqueous medium.

Processes for the fermentation of the aforementioned fungal strains are known in principle to the person skilled in the art, for example from EP 0 271 907 A2, EP 0 504 673 A1, DE 40 12 238 A1, WO 03/016545 A2, and "Udo Rau, "Biosynthese, Produktion und Eigenschaften von extrazellulären Pilz- Glucanen [Biosynthesis, production and properties of extracellular fungal glucans]", Postdoctoral thesis, Technical University of Braunschweig, 1997". These documents also each describe suitable aqueous media or nutrient media.

The fungal strains are preferably cultivated in an aqueous medium at a temperature in the range from 15°C to 40°C, particularly preferably in the range from 25 to 30°C, preferably with aeration and agitation, for example using a stirrer.

In order to ensure an efficient process for the fermentation of the fungal strains, the fermentation takes place in a cascade of tanks. In this connection, in a preceding tank, an amount of fungal strains and thus also a volume of the aqueous medium is produced that is adequate to bring about as rapid as possible a fermentation in the subsequent tank.

According to an exemplary embodiment the high-shear mixer is a rotor-stator mixer having a rotor and a stator.

Thus, it is possible to provide an efficient shear process, in particular when transferring the mixture from the first tank to the second tank. A rotor-stator mixer has a high through flow capacity and a reliable shearing characteristic. Further a rotor-stator mixer allows an in-line process, which means that the mixture once passes the shearing geometry and then is sufficiently sheared. According to an exemplary embodiment the rotor-stator mixer(s) include types of for example, toothed-rim dispersers, annular-gap mills and colloid mills.

According to an exemplary embodiment the rotor-stator mixer is a toothed-rim dispersing machine.

Thus, it is possible to provide a reliable shearing geometry. If the agglomerates after the shearing process are too small, or are destroyed during shearing, the fermentation process also may be less efficient. A toothed-rim dispersing

machine allows a sufficient shearing without too much destroying the agglomerates.

According to an exemplary embodiment rotor-stator mixers are used which have means for generating cavitation forces. Means of this type may be elevations on the rotor and/or stator side which protrude into the mixing chamber and have at least one face where the normal has a tangential portion, such as, for example, pins, teeth or blades or coaxial rings having radial disposed slits.

According to an exemplary embodiment the rotor-stator mixer has on the rotor side at least one rotational symmetrically disposed toothed rim and/or at least one rotational symmetrically disposed ring having radial slits (space widths). Apparatuses of this type are also referred to as toothed-rim dispersers or toothed-rim dispersing machines. In particular, the rotor-stator mixer has, on both the rotor side and the stator side, at least one rotational symmetrically disposed toothed rim and/or ring with radial slits (space widths), where the toothed rims/rings disposed on the rotor and stator side are arranged coaxially and mutually intermesh to form an annular gap.

According to an exemplary embodiment the rotor-stator mixer has a construction which corresponds to a stand with annular ridges with slits cut therein and a rotor with annular ridges with slits cut therein which are arranged concentrically and which are arranged at a distance such that they intermesh with one another. With this rotor-stator mixer the aqueous media or mixtures are fed into the middle section between the stand/stator and the rotor, while the rotor is left to rotate, so that it presses the aqueous media or mixture through the slit and the gap middle the medium section in the direction of the perimeter.

According to an exemplary embodiment at least one of the rotor and the stator of the rotor-stator mixer has at least two concentric toothed-rims and the other of the rotor and the stator has at least one toothed rim, wherein the at least one toothed-rim of the other of the rotor and the stator concentrically interleaves with the at least two concentric toothed-rims, wherein the first aqueous medium passes through the interleaved toothed-rims.

Thus, it is possible to have a defined flow path through the high shear mixer. The shearing geometry may have a geometry, which allows a well defined shearing process, the result of which are agglomerates having a suitable size distribution.

According to an exemplary embodiment the at least two concentric toothed-rims of one of the rotor and the stator and the at least one toothed rim of the other of the rotor and the stator have an equidistant tooth geometry, wherein the distance between adjacent teeth of the respective outer toothed-rim is larger than the distance between adjacent teeth of the respective inner toothed-rim, wherein the first aqueous medium passes through the interleaved toothed-rims in a direction of ascending teeth distance.

Thus, it is possible to provide a quasi stepped shearing process within the rotor-stator mixer. As all toothed rims of the rotor rotate with the same number of revolutions per minute, the track speed of the radial outer rims is higher than the track speed of the radial inner rims. When providing a larger tooth distance at radial outer rims, the shearing effect may be adapted and a destruction of the agglomerates in particular at the outer rims can be avoided. Further, a clogging effect in the flow path of the high shear mixer can be avoided.

According to an exemplary embodiment the first mixture passes through a gap in radial direction, which gap in a radial direction is formed by the concentrically interleaving at least two concentric toothed-rims of one of the rotor and the stator and the at least one toothed-rim of the other of the rotor and the stator, wherein the gap between an outer diameter of a toothed rim and an inner diameter of a radial outwardly adjacent toothed-rim has a width between 0.2mm and 2.0mm, preferably 0.4mm and 1.2mm, more preferably between 0.8mm and 0.9mm.

Thus, the size of the agglomerates when leaving the shear mixer may be in a particular size range. This size range may lead to a particularly efficient fermentation process in the subsequent tank behind the high shear mixer.

According to an exemplary embodiment the first mixture dwells for between 0.01s and 0.004s, preferably between 0.02 and 0.07s, more preferably 0.01s +/- 0.005s while passing the least two concentric toothed-rims of one of the rotor and the stator and the at least one toothed-rim of the other of the rotor and the stator.

Thus, the shearing process can be optimized. The longer the mixture remains in the shear mixer the longer the shearing process takes. On the other hand a quick passing may destroy the agglomerates or may lead to an agglomerate size being too large for an efficient fermentation process.

According to an exemplary embodiment edges of teeth along a flow path through the shearing geometry have rounded edges with a radius of at least 0.2 mm, in particular more than 3mm.

Thus, the agglomerates do not collide with sharp edges and do not underlay a cutting. The rounded edges allow a shearing of the agglomerates and at the same time preserve the agglomerates from unintended cutting within the high shear mixer. Further, it can be avoided that particular agglomerates or parts thereof or other residuals remain in the shearing geometry.

According to an exemplary embodiment the rotor rotates at a speed relative to the stator between 250 and 7200, preferably between 1800 and 6000, more preferably between 4000 and 4500 revolutions per minute.

Thus, the shearing process, in particular in view of the above described geometries and dimensions, is efficient and also the subsequent fermentation process is efficient.

According to an exemplary embodiment the rotor rotates at a peripheral/track speed of the outmost toothed

rim of between 2 m/s and 60 m/s, preferably between 15 m/s and 50 m/s, more preferably between 35 m/s and 45 m/s.

Thus, the maximum force may be kept in a particular range within the high shear mixer. In particular the agglomerates do not suffer from a too high tension treatment possibly leading to a destruction of the agglomerates.

According to an exemplary embodiment the proportion of the volume of the first mixture to the volume of the second tank is in the range between ≥ 1 % to ≤ 20%.

Thus, the fermentation process can be made more efficient. According to an exemplary embodiment the proportion of the volume of the first mixture to the volume of the second tank is in the range between ≥ 2.5% to ≤ 15%.

According to an exemplary embodiment the at least one beta-glucan is selected from the group consisting of *Schizophyllan* and *Scleroglucan,* wherein the *Schizophyllan* or *Scleroglucan* are obtained by fermentation of fungal strains. According to an exemplary embodiment the fungal strains are *Schizophyllum commune* or *Sclerotium rolfsii*.

According to an exemplary embodiment the process for the fermentation of fungal strains is carried out in a tank cascade which further comprises a third tank with a third volume, and the process further comprises at least the steps of

d) transferring the second mixture to the third tank, and

e) fermenting the fungal strains in the second mixture in a third aqueous medium in the third tank, wherein the proportion of the second mixture to the volume of the third tank is in the range between ≥ 0.1% to ≤ 50%.

Thus a further cascade step can be provided. The entire fermentation process can be improved. Three fermentation steps with two interleaved shearing processes allow a more controlled process for fermentation of fungal stains. The entire process can be sped up and the efficiency can be increased.

According to an exemplary embodiment the second mixture in step d) is passed through at least one high-shear mixer, the high-shear mixer has a shearing geometry, such that the entire second mixture entirely passes through the shearing geometry of the at least one high-shear mixer.

According to an exemplary embodiment the proportion of the second mixture to the volume of the third tank is in the range between ≥ 1% to ≤ 20%, in particular in the range between ≥ 2.5% to ≤ 15%.

The high-shear mixer is preferably a high-shear mixer selected from the group consisting of rotor-stator mixers and high-pressure homogenizers, as described above.

According to an exemplary embodiment, there is provided a process for the fermentation of fungal strains which secrete glucans with a β-1,3-glycosidically linked main chain and side groups β-1,6-glycosidically linked thereto in a cascade of tanks comprising at least a first tank with a first volume, a second tank with a second volume, a third tank with a third volume and a fourth tank with a fourth volume, comprising at least the steps

a) fermentating the fungal strains in a first aqueous medium in the first tank and the volume of the first aqueous medium resulting in a first mixture,

b) transferring the first mixture to the second tank,

c) fermenting the fungal strains in the first mixture in a second aqueous medium in the second tank and the volume of the second aqueous medium resulting in a second mixture,

d) transferring the second mixture to the third tank,

e) fermenting the fungal strains in the second mixture in a third aqueous medium in the third tank and the volume of the third medium resulting in a third mixture,

f) transferring the third mixture to the fourth tank, and

g) fermenting the fungal strains in the third mixture in a fourth aqueous medium in the fourth tank,

where the proportion of the volume of the first mixture to the volume of the second tank is in the range between ≥ 0.1% to ≤ 50%, the proportion of the volume of the second mixture to the volume of the third tank is in the range between ≥ 0.1 % to ≤ 50%, and the proportion of the volume of the third mixture to the volume of the fourth tank is in the range between ≥ 0.1 % to ≤ 50%, wherein the first mixture in step b) is passed through at least one high-shear mixer and/or the second mixture in step d) is passed through at least one high-shear mixer and/or the third mixture in step f) is passed through at least one high-shear mixer, wherein at least one of the high shear mixers has a shearing geometry, such that the entire respective mixture entirely passes through the shearing geometry of the at least one high-shear mixer.

According to an exemplary embodiment the proportion of the volume of the first aqueous medium to the volume of the second tank is in the range between ≥ 1% to ≤ 20%, particularly in the range between ≥ 2.5% to ≤ 15%.

According to an exemplary embodiment the proportion of the volume of the second aqueous medium to the volume of the third tank is in the range between ≥ 1 % to ≤ 20%, particularly in the range between ≥ 2.5% to ≤ 15%.

According to an exemplary embodiment the proportion of the volume of the third aqueous medium to the volume of the fourth tank is in the range between ≥ 1% to ≤ 20%, particularly in the range between ≥ 2.5% to ≤ 15%.

According to an exemplary embodiment the fermentation is performed such that the concentration of the glucans

to be prepared in the aqueous medium at the end of the fermentation process in the last tank of the reactor cascade is at least 3 g/l. The upper limit is not limited in principle. It results from what viscosity can still be handled in the tank used.

According to an exemplary embodiment the high-shear mixers used in the transferring step have an identical or different design in the individual steps. According to an exemplary embodiment the rotor-stator mixers used in the transferring step in the individual steps have an identical or different design.

Rotor-stator mixers may also comprise in principle all dynamic mixer types in which a highspeed rotor, which may be a rotational symmetrical, cooperates with a stator to form one or more processing regions which are essentially in the shape of an annular gap. In these processing regions, the mixing material is subjected to severe shear stresses, while the high level of turbulence which often prevails within the annular gaps likewise promotes the mixing operation.

In a further embodiment, the high-shear mixer is a high-pressure homogenizer. In such a mixer, the aqueous medium is forced through a small opening under high pressure.

Preferably, the high pressure is in the range from 100 bar to 2000 bar, particularly preferably in the range from 200 bar to 1000 bar.

Preferably, the small opening has a diameter in the range from 0.5 to 2.5 cm, particularly preferably in the range from 0.8 to 2.0 cm.

In a further embodiment, the fermentation broth comprising at least one beta-glucan and biomass (fungal cells with or without cell constituents) at the end of the fermentation process is filtered.

Preferably, the content of the fermentation tank after the fermentation is filtered with the use of asymmetrical filter membranes or symmetrical filter membranes.

Alternatively, the fermentation broth is removed continuously or from time to time from the plant via a side stream and an aqueous solution comprising at least one beta-glucan is separated off therefrom by crossflow microfiltration. The remaining aqueous fermentation broth in which the biomass has a higher concentration than beforehand can be at least partly recycled to the fermentation container.

The crossflow microfiltration process is known in principle to the person skilled in the art and is described, for example, in "Melin, Rautenbach, Membranverfahren, Springer-Verlag, 3rd edition, 2007, page 309 to page 366". Here, "microfiltration" is understood by the person skilled in the art as meaning the removal of particles having a size of from about 0.1 $\mu$m to about 10 $\mu$m.

In the crossflow filtration, a stream of the liquid to be filtered is applied, for example, by a suitable circulation pump, parallel to the surface of the membrane used as filtration material. A liquid stream therefore continuously flows over the filter membrane, and the formation of deposits on the membrane surface is prevented or at least reduced thereby. In principle, all types of pump are suitable as the pump. Owing to the high viscosity of the medium to be transported, however, in particular positive displacement pumps and very particularly eccentric screw pumps and rotary piston pumps have proven useful.

Preferably, asymmetrical filter membranes or symmetrical tubular membranes are used for the crossflow microfiltration. Asymmetrical filter membranes consist of at least two different layers having different pore size, i.e. of at least one support layer and one separating layer. The support layer is comparatively thick and has comparatively large pores. It imparts the mechanical strength to the filter membrane. At least one separating layer having finer pores than the pores of the support layer is applied to the support layer. For example, mercury porosimetry can be used in a manner known in principle for measuring the pore sizes. Optionally, one or more intermediate layers may also be arranged between the separating layer and the support layer.

The asymmetrical membranes may be, for example, metallic membranes or ceramic membranes. The asymmetrical membranes used are preferably asymmetrical ceramic membranes. Details of asymmetrical ceramic membranes are described, for example, in "Melin, Rautenbach, Membranverfahren, Springer-Verlag, 3rd edition, 2007, page 51 to page 52".

Symmetrical tubular membranes are tubular membranes which have a pore distribution which is essentially constant over the entire cross section of the membrane wall. Symmetrical tubular membranes are known to those skilled in the art and are described, inter alia, in "Melin, Rautenbach, Membranverfahren, Springer-Verlag, 3rd edition, 2007, page 20".

The good quality of the aqueous solution comprising at least one beta-glucan may be evident from the good filtration properties, which are expressed by the low filtration ratio (FR value). In a preferred embodiment, the FR value of the product may be preferably in the range of ≥10 to ≤1.8, more preferably in the range of ≥10 to ≤1.5, even more preferably in the range of ≥10 to ≤1.3.

In another preferred embodiment, the yield of at least one beta-glucan after filtration, i.e. the amount of at least one beta-glucan which can be recovered from the fermentation broth, based on the amount of at least one beta-glucan present in the fermentation broth prior to filtration, is preferably in the range from ≥25% to ≤97%, more preferably in the range from ≤30% to ≥95% and most preferably in the range from ≤50% to ≤93%.

The aqueous solution containing at least one beta-glucan may be further worked up and concentrated in order to obtain the at least one beta-glucan in highly concentrated form. In one embodiment, the aqueous solution comprising

at least one beta-glucan can be brought into contact with at least one precipitating agent to obtain at least one precipitated beta-glucan in a solvent mixture comprising water and the at least one precipitating agent. Preferably, the at least one precipitating agent is selected from the group consisting of low boiling liquids, high boiling liquids and mixtures thereof. Examples of low boiling liquids are formates like methyl formate, acyclic ethers like dimethoxymethane, cyclic ethers like tetrahydrofuran, 2-methyl-1,2-dioxalane, carboxylic acid esters like acetic acid ethyl ester, alcohols like methanol, ethanol, isopropanol or propanol, ketones like acetone or methylethylketone, or mixtures of at least two of them. Examples of high boiling liquids are polyethylene glycols having molecular weights preferably in the range of 10 to 200 kD, more preferably in the range of 15 to 120 kD, polypropylene glycols having molecular weights in the range of 5 to 100 kD, more preferably 10 to 30 kD, or mixtures of at least two of them. The at least one precipitating agent is generally added to the aqueous solution comprising at least one beta-glucan, so that the volume ratio of the precipating agent to the aqueous solution is in the range of preferably 0.1:1 to 20:1, more preferably 0.2:1 to 2:1, most preferably 0.2:1 to 1.5:1, in each case based on the total mixture that is obtained.

The at least one precipitated beta-glucan can be separated from the solvent mixture comprising water and the at least one precipitating agent to obtain a precipitated beta-glucan in highly concentrated form. The separation can in general be conducted by any methods known to the skilled artisan, for example, *inter alia*, centrifugation, sedimentation, flotation and filtration.

The beta-glucan such as schizophyllan which is obtained according to the inventively claimed method may be further modified after filtration and optionally concentration. The beta-glucan such as schizophyllan may be converted by oxidation, enzyme conversion, acid hydrolysis, heat and/or acid dextrinization or shear. The beta-glucan such as schizophyllan can also be chemically, enzymatically or physically modified. Suitable chemical derivatives of schizophyllan include esters, such as the acetate and half esters, such as the succinate, octenyl succinate and tetradecenyl succinate, phosphate derivatives, ethers such as hydroxyalkyl ethers and cationic ethers, or any other derivatives or combinations thereof. Modification may also be chemical crosslinking. Crosslinking agents that are suitable for use herein include phosphorus oxychloride, epichlorohydrin, sodium trimetaphosphate and adipic acid/ acetic acid mixed anhydrides.

It should be noted that the above features may also be combined. The combination of the above features may also lead to synergetic effects, even if not explicitly described in detail.

These and other aspects of the present invention will become apparent from and elucidated with reference to the embodiments described hereinafter.

## Brief Description of the Drawings

Exemplary embodiments of the present invention will be described in the following with reference to the following drawings.

Fig. 1. illustrates a two-step fermentation process with an interleaved shearing process according to an exemplary embodiment.

Fig. 2. illustrates a three-step fermentation process with two interleaved shearing processes according to an exemplary embodiment.

Fig. 3. illustrates a four-step fermentation process with three interleaved shearing processes according to an exemplary embodiment.

Fig. 4. illustrates a cross sectional view of a high shear mixing geometry according to an exemplary embodiment.

Fig. 5a. illustrates a top view of one of a rotor and a stator of a high shear mixer according to an exemplary embodiment.

Fig. 5b. illustrates a top view of the other of a rotor and a stator of a high shear mixer according to an exemplary embodiment in view of Fig. 5a.

Fig. 6. illustrates a detailed cut out of a cross sectional view of a high shear mixer geometry according to an exemplary embodiment.

Fig. 7 illustrates an exemplary space-time-yield over time chart for a laboratory fermenter with/without morphology control.

Fig. 8 illustrates an exemplary space-time-yield over time chart for a pilot plant fermenter with/without morphology control.

**Detailed Description of Exemplary Embodiments**

Fig. 1 illustrates a two-step fermentation process with an interleaved shearing process according to an exemplary embodiment. Fig. 1 in particular illustrates the general set-up of the tank and shear mixer structure. A first tank K1 having a first tank volume VK1 and receives a first aqueous mediumM1. A fermentation of fungal strains takes place in the first aqueous medium M1, resulting in a first mixture S1. During fermentation, the fungal strains form agglomerates. The first mixture S1 including the agglomerates of fungal strains is transferred to a second tank K2 having a second tank volume VK2. A second aqueous medium M2 may be added to the first mixture S1, so that a further fermentation of fungal strains in the first mixture in a second aqueous medium in the second tank takes place, resulting in a second mixture S2. As the agglomerates in the first mixture before being transferred from the first tank K1 to the second tank K2 are large and do not allow an efficient fermentation process in the second tank, the first mixture S1 flows through a high-shear mixer 1 being arranged between the first tank K1 and the second tank K2. The proportion of the volume of the first mixture VM1 to the volume of the second tank VK2 is in the range between 0.1% to 50%. The high-shear mixer 1 is of a type in view of a shearing geometry, such that the entire first mixture S1 entirely passes through the shearing geometry of the high-shear mixer 1. The detailed geometry of the high-shear mixer is later described with respect to Figs. 4, 5a, 5b and 6.

Fig. 2 illustrates a three-step fermentation process with two interleaved shearing processes according to an exemplary embodiment. Fig. 2 illustrates a first tank K31 with a first tank volume VK31. A first aqueous medium M31 is in the first tank volume VK31. A fermentation of fungal strains takes place in the first aqueous medium M31 in the first tank volume VK31, resulting in a first mixture S31. The first mixture S31 is transferred to the second tank K32 having a second tank volume VK32. An aqueous medium M32 is added to the first mixture S31 in the second tank volume VK32, so that a fermentation of fungal strains in the first mixture in the second aqueous medium M32 takes place. As the fungal strains form agglomerates during fermentation in the first tank, the size of the agglomerates should be reduced, e.g. by a shearing process by a high-shear mixer 1 being arranged between the first tank K31 and the second tank K32. Consequently, the first mixture S31 flows through the high-shear mixer 1 and will be sheared, and then enters the second tank K32. The proportion of the volume of the first mixture VM31 to the volume of the second tank K32 may be in the range between 0.1% to 50%. The first mixture S31 entirely passes through the high-shear mixer 1, wherein the high-shear mixer 1 has a shearing geometry, such that the entire first mixture S31 entirely passes through the shearing geometry of the high-shear mixer 1. This means that the shear mixer has a flow through geometry. After a further fermentation of fungal strains in the first mixture and the second aqueous medium M32, the resulting second mixture S32 will be transferred to a third tank K33. The second mixture S32 for this purpose passes a further high-shear mixer 1 so that the again formed agglomerates will be again sheared before entering the third tank K33. In the third tank, the second mixture S32 will be added to a third aqueous medium M33, so that a further fermentation can take place in the volume VK33 of the third tank K33.

Fig. 3 illustrates a four-step fermentation process with three interleaved shearing processes according to an exemplary embodiment. Fungal strains in a first aqueous medium M41 in a volume VK41 in a first tank K41 are fermented, resulting in a first mixture S41. During fermentation process, fungal strains form agglomerates, which possibly do not allow an efficient further fermentation, so that the agglomerates should be sheared before starting a further fermentation in the second tank K42 having a second tank volume VK42. Thus, the first mixture S41 is transferred to the second tank K42 and during transfer passes the high-shear mixer 1 between the first tank K41 and the second tank K42. The first mixture S41 including the sheared agglomerates will be added to a second aqueous medium M42, so that a further fermentation may take place, resulting in a second mixture S42. The second mixture S42 will then be transferred to a third tank K43 having a third tank volume VK43. The second mixture S42 passes a high-shear mixer 1, so that the agglomerates being formed during the second fermentation will be sheared. The second mixture in the third tank K43 will be added to a third aqueous medium M43. Thus, a third fermentation process may take place in the tank volume VK43, resulting in a third mixture S43. Also the third mixture S43 may include agglomerates which may decrease efficiency of a further fermentation. Therefore, the third mixture S43 also passes a high-shear mixer 1 before entering a fourth tank K44 having a fourth tank volume VK44. In the fourth tank volume VK44, the third mixture S43 will be added to a fourth aqueous medium M44. A further fermentation may take place in the fourth tank volume VK44.

It should be noted, although not explicitly described, that also a fermentation process can be provided having more than four steps as described above with respect to Fig. 3. It should be noted that the high-shear mixers 1 between two respective tanks may have different specifications according to the expected structure of the agglomerates in the respective tank after fermentation.

Further, it should be noted that in all three embodiments as described above Figs. 1, 2 and 3, the proportion of the volume of the first mixture VM1, VM31, VM41, to the volume of the second tank VK2, VK32, VK42 may be in the range between 0.1% and 50%. Further, it should be noted, that for all three above described embodiments with respect to Fig. 1, 2 and 3, the proportion of the volume of the first mixture VM1, VM31, VM41, to the volume of the second tank VK2, VK32, VK42 may be in a range between 1% and 20%.

Further, it should be noted that for the embodiments described with respect to Fig. 2 and 3, i.e. the three-step

fermentation process and the four-step fermentation process, the proportion of the second mixture S32, S42 to the volume of the third tank VK33, VK43 may be in a range between 0.1% and 50%, and in particular between 1% and 20%.

Additionally, the proportion of the third mixture S43 to the volume of the fourth tank VK44 in the embodiment described with respect to Fig. 3 may be in a range between 0.1% to 50%, and in particular between 1% and 20%.

Fig. 4 illustrates a cross-sectional view of a high-shear mixing geometry according to an embodiment. A high-shear mixer according to the illustrated embodiment of Fig. 4 comprises a rotor 10 and a stator 20. The rotor has a first toothed-rim 11 having a plurality of teeth 13. The rotor 10 further has a second toothed-rim 12 also comprising a plurality of teeth 13. The stator 20 also has a first toothed-rim 21 having a plurality of teeth 23. Further, the stator has a second toothed-rim 22 also having a plurality of teeth 23. The teeth of each of the toothed-rims 11, 12, 21, 22 are arranged along a circuit being concentric to the rotational axis of the high-shear mixer 1. The toothed-rims of the rotor 11, 12 and the toothed-rims of the stator 21, 22 interleave so as to form a gap 2 between the teeth as such, and the rotor and stator body, respectively. The mixture to be sheared will be fed through for example a through-hole of the rotor 10 and flows along the double arrows in Fig. 4, so that the mixture S1 will be sheared between teeth of adjacent rims. It should be noted, that the feeding of the mixture S1 can also take place through a through-hole of the stator, although this specification is not explicitly illustrated in Fig. 4. Further, it should be noted that the number of toothed-rims of the rotor as well as the stator may be more than two.

Fig. 5a illustrates a top view of one of a rotor and a stator of a high-shear mixer according to an embodiment. In particular, Fig. 5a illustrates a rotor 10 having a first toothed-rim 11 including a plurality of teeth 13. Further, a second toothed-rim 12 is provided on the rotor. It should be noted, that the configuration illustrated in Fig. 5a may also be a configuration for a stator. The teeth 13 of the first and second toothed-rims 11, 12 may be different as well as the width of the teeth and the width of the gap there between in a circumferential direction.

Fig. 5b illustrates a top view of the other of a rotor and a stator of a high-shear mixer according to an embodiment in view of Fig. 5a, and in particular a stator 20. The stator 20 has at least one rim 21 having a plurality of teeth 23. As can be seen by the dashed lines between Fig. 5a and Fig. 5b, the toothed-rims of the rotor 10 and the stator 20 interleave when being coupled as illustrated in Fig. 4.

Fig. 6 illustrates a detailed cut-out of a cross-sectional view of a high-shear mixer geometry according to an exemplary embodiment. Fig. 6 illustrates the rotor 10 and the stator 20 with respective teeth of a toothed-rim. It should be noted that the rotor 10 and/or the stator 20 may have a further toothed-rim with a similar geometry. The teeth 13 and 23 of the toothed-rims 11 and 21 of the rotor 10 and the stator 20, respectively have rounded edges. The edges have a radius R so as to provide a smooth transition between the teeth and the stator body or the teeth and the rotor body, as well as between the teeth and the gap 2. The rounded edges 14, 24 result in a reduced impact to the agglomerates of the mixture, so that the agglomerates are not cut or destroyed by sharp edges of the teeth 13, 23, which will result in a deteriorated fermentation process. It should be noted that rounded edges may be provided in particular at edges between teeth of adjacent rims. Further, rounded edges can also be provided between adjacent teeth of a single rim. The radius R of teeth of adjacent rims may be adapted to each other so as to have a more or less continuous width of the gap 2.

**Examples**

The Schizophyllum commune strain used is laid open in EP 0 504 673.
Suitable nutrient media for the precultures and main cultures and cultivation conditions can be found for example in the patent EP 504 6073, EP 0 271 907 and "Process and molecular data of branched 1,3-β-D-glucans in comparison with Xanthan, U. Rau, R. -J. Müller, K. Cordes, J. Klein, Bioprocess Engineering, 1990, Volume 5, Issue 2, pp 89-93" and "Udo Rau, "Biosynthese, Produktion und Eigenschaften von extrazellulären Pilz-Glucanen [Biosynthesis, production and properties of extracellular fungal glucans]", Postdoctoral thesis, Technical University of Braunschweig, 1997".

Nutrient medium used: 30 g/l glucose, 3 g/l yeast extract, 1 g/l $KH_2PO_4$, 0.5 $MgSO_4 * 7 H_2O$

1. Preculture

Strain maintenance and cultivation of the biomass are described for example in "Oxygen controlled batch cultivations of Schizophyllum commune for enhanced production of branched β-1,3-glucans, U. Rau, C. Brandt Bioprocess Engineering September 1994, Volume 11, Issue 4, pp 161-165". The ratio of the volumes upon transfer was about 5%.

All of the tanks of the preculture were operated at a constant speed and gassing rate so that the $pO_2$ was always above 60%. The duration of the precultures was chosen such that the glucose did not drop below 5 g/l.

2. Main culture

The main culture was carried out according to the process described in the literature under oxygen-limiting

conditions. The procedure for the main culture is described for example in "Oxygen controlled batch cultivations of Schizophyllum commune for enhanced production of branched β-1,3-glucans, U. Rau, C. Brandt Bioprocess Engineering September 1994, Volume 11, Issue 4, pp 161-165", "Udo Rau, "Biosynthese, Produktion und Eigenschaften von extrazellulären Pilz-Glucanen [Biosynthesis, production and properties of extracellular fungal glucans]", Postdoctoral thesis, Technical University of Braunschweig, 1997" and "Process and molecular data of branched 1,3-β-D-glucans in comparison with Xanthan, U. Rau, R. -J. Müller, K. Cordes, J. Klein, Bioprocess Engineering, 1990, Volume 5, Issue 2, pp 89-93",

3. Transfer of the preculture to the main culture with rotor-stator mixer

The increase in volumetric productivity in the main culture through the use of a toothed-wheel pump in the bypass, as described in DE 4012238 A1, could not be recreated. The opposite effect was observed in experiments that the recirculation via a bypass, as described in DE 4012238 A1, significantly reduces the volumetric productivity in the main culture.

Surprisingly, it was found that using a continuously operated rotor-stator mixer when transferring the preculture to the main culture leads to a significant increase in the STY. In this example, a rotor-stator mixer from Cavitron was used, bench instrument CD 1000 equipped with a chamber system, operated at 5 - 20 l/min, peripheral speed: 3-50 m/s.

The rotor-stator mixer was incorporated into the pipeline of the last tank of the preculture to the main culture tank in the reactor cascade and steam-sterilized prior to insertion in order to permit aseptic operation.

4. Determination of the space-time yield

The space-time yield (STY), also called volumetric productivity, was determined by measuring the glucan concentration in a sample taken after a runtime of 72 h using a method described in the literature. The measured concentration divided by the runtime until the sample was taken (72 h) gives the space-time yield. For the purposes of simplification, relative STY are shown. The STY which were achieved without using a high-shear mixer were set as 100%.

5. Determination of the filtration ratio (FR value)

Principle of measurement:

In the determination of the filtration ratio (FR value), the amount of filtrate which runs through a defined filter is determined as a function of time. The FR value is determined according to the following formula (I)

$$FR = (t_{190g} - t_{170g}) / (t_{70g} - t_{50g}) \qquad (I),$$

where the variables and the equation have the following meaning:

$t_{190g}$ = time in which 190 g of filtrate are obtained,
$t_{170g}$ = time in which 170 g of filtrate are obtained,
$t_{70g}$ = time in which 70 g of filtrate are obtained,

$t_{50g}$ = time in which 50 g of filtrate are obtained.

Thus, in each case the time span which is required for in each case 20 g of filtrate to flow through is determined, i.e. at a early time and at a late time in the filtration process, and the quotient is calculated from the two time spans. The larger the FR value, the more greatly is the filtration velocity slowed down with increasing duration of the filtration process. This indicates increasing blockage of the filter, for example by gels or particles.

The FR value is determined by the following method:

5.1. Equipment

a) Sartorius pressure filtration apparatus 16249; filter diameter 47 mm; with 200 ml digestion cylinder (∅i = 41 mm)
b) Isopore membrane 1.2 μm; ∅ 47 mm; No. RTTP04700 available from Merck Millipore
c) Balance

5.2. Preparation of the glucan solution

First, 50 g of a mixture of the glucan solution obtained from the experiments and water is prepared, i.e. in a ratio such that the concentration of the glucan is 1.75 g/l. The mixture is stirred for 10 min and checked visually for homogeneity. If the mixture is still inhomogeneous, further stirring is effected until the mixture is homogeneous. The mixture is then made up to a total amount of 250 g with 200 g of ultrapure water. Thereafter, stirring is effected for at least 1 h for homogenization, after which the pH is adjusted to 6.0 with 0.1 M NaOH and stirring is then effected again for 15 min. The pH of 6.0 is checked again. The final concentration of the glucan in the mixture is 0.35 g/l.

5.3. Carrying out the filtration test

The filtration test is effected at room temperature (T = 25°C) at a pressure of 1.0 bar (compressed air or $N_2$).

- place coarse support grid on the sieve tray
- place fine support grid on the sieve tray
- place membrane filter on top
- insert seal (O-ring)
- screw sieve tray and outlet tap to the cylinder
- close outlet tap
- introduce 220 g (about 220 ml) of solution
- screw upper cover to cylinder
- clamp on inlet air tube
- check pressure and adjust to 1.0 bar
- place beaker on the balance under the filtration apparatus. Press tare.
- open outlet tap
- the test is stopped when no more filtrate emerges.

By means of the balance, the amount of filtrate is determined as a function of time. The mass indicated in each case can be read visually but of course also automatically and evaluated.

Fig. 7 illustrates an exemplary relative space-time-yield over time chart for a laboratory fermenter with/without morphology control. As can be seen from Fig. 7, a laboratory fermenter with morphology control has a higher relative space-time-yield compared to a laboratory fermenter without a morphology control. Thus, the efficiency of the laboratory fermenter with morphology control is higher than a laboratory fermenter without a morphology control. In particular, Fig. 7 shows the comparison of the relative STY for production on a laboratory scale (21 l) with a three-stage preculture. It can be seen that the STY is significantly increased in the case of morphology control to avoid pellet or agglomerate formation.

Fig. 8 illustrates an exemplary relative space-time-yield over time chart for a pilot plant fermenter with/without morphology control. As can be seen from Fig. 8, the relative space-time-yield of a pilot plant scale fermenter with morphology control is a little bit higher than a relative space-time-yield of a pilot plant scale fermenter without morphology control. In particular, Fig. 8 shows the comparison of the relative STY for the production on a pilot-plant scale (3 $m^3$) with a three-stage preculture. It can be seen that the STY is significantly increased in the case of morphology control to avoid pellet/agglomerate formation.

Reference list:

| | |
|---|---|
| 1 | high shear mixer |
| 2 | gap |
| 10 | rotor |
| 11 | toothed rim of rotor |
| 12 | toothed rim of rotor |
| 13 | tooth/teeth of toothed rim of rotor |
| 14 | edge of tooth |
| 20 | stator |
| 21 | toothed rim of stator |
| 22 | toothed rim of stator |
| 23 | tooth/teeth of toothed rim of stator |

| | |
|---|---|
| 24 | edge of tooth |
| K1 | first tank |
| K2 | second tank |
| K31 | first tank |
| K32 | second tank |
| K33 | third tank |
| K41 | first tank |
| K42 | second tank |
| K43 | third tank |
| K44 | fourth tank |
| M1 | first aqueous medium |
| M2 | second aqueous medium |
| M31 | first aqueous medium |
| M32 | second aqueous medium |
| M33 | third aqueous medium |
| M41 | first aqueous medium |
| M42 | second aqueous medium |
| M43 | third aqueous medium |
| M44 | fourth aqueous medium |
| S1 | first substance |
| S2 | second substance |
| S31 | first mixture |
| S32 | second mixture |
| S41 | first mixture |
| S42 | second mixture |
| S43 | third mixture |
| VK1 | first tank volume |
| VK2 | second tank volume |
| VK31 | first tank volume |
| VK32 | second tank volume |
| VK33 | third tank volume |
| VK41 | first tank volume |
| VK42 | second tank volume |
| VK43 | third tank volume |
| VK44 | fourth tank volume |
| VM1 | volume of first aqueous medium |
| VM2 | volume of second aqueous medium |
| VM31 | volume of first aqueous medium |
| VM32 | volume of second aqueous medium |
| VM33 | volume of third aqueous medium |
| VM41 | volume of first aqueous medium |
| VM42 | volume of second aqueous medium |
| VM43 | volume of third aqueous medium |
| VM44 | volume of fourth aqueous medium |

## Claims

**1.** A process for the fermentation of fungal strains which secrete glucans with a β-1,3-glycosidically linked main chain and side groups β-1,6-glycosidically bonded thereto, in a cascade of tanks comprising at least a first tank (K1, K31) with a first volume (VK1, VK 31) and a second tank (K2, K32) with a second volume (VK2, VK32), comprising at least the steps

a) fermentation of the fungal strains in a first aqueous medium (M1, M31) in the first tank (K1, K31) and the volume of the first aqueous medium (VM1, VM31), resulting in a first mixture (S1, S31),
b) transfer of the first mixture (S1, S31) to the second tank (K2, K32), and
c) fermentation of the fungal strains in the first mixture (S1, S31) in a second aqueous medium (M2, M32) in the second tank (K2, K32) and the volume of the second aqueous medium (VM2, VM32), resulting in a second

mixture (S2, S32),

where the proportion of the volume of the first mixture (VM1, VM31) to the volume of the second tank (VK2, VK32) is in the range between ≥ 0.1% to ≤ 50% and where the first mixture (S1, S31) in step b) is passed through at least one high-shear mixer, the high-shear mixer (1) has a shearing geometry, such that the entire first mixture (S1, S31) entirely passes through the shearing geometry of the at least one high-shear mixer.

**2.** The process according to claim 1, wherein the high-shear mixer (1) is a rotor-stator mixer having a rotor (10) and a stator (20).

**3.** The process according to claim 2, wherein the rotor-stator mixer is a toothed-rim dispersing machine.

**4.** The process according to any one of claims 2 and 3, wherein at least one of the rotor (10) and the stator of the rotor-stator mixer has at least two concentric toothed-rims (11, 12) and the other of the rotor and the stator (20) has at least one toothed rim (21, 22), wherein the at least one toothed-rim of the other of the rotor and the stator concentrically interleaves with the at least two concentric toothed-rims, wherein the first aqueous medium (M1, M31) passes through the interleaved toothed-rims.

**5.** The process according to claim 4, wherein the at least two concentric toothed-rims (11, 12) of one of the rotor (10) and the stator and the at least one toothed rim (21, 22) of the other of the rotor and the stator (20) have an equidistant tooth geometry and wherein the distance between adjacent teeth (13) of the respective outer toothed-rim (11) is larger than the distance between adjacent teeth (23) of the respective inner toothed-rim (21), wherein the first aqueous medium M1 passes through the interleaved toothed-rims in a direction of ascending teeth distance.

**6.** The process according to any one of claims 4 and 5, wherein the first mixture (S1) passes through a gap (2) in radial direction, which gap in a radial direction is formed by the concentrically interleaving at least two concentric toothed-rims (11, 12) of one of the rotor (10) and the stator and the at least one toothed-rim (21, 22) of the other of the rotor and the stator (20), wherein the gap (2) between an outer diameter of a toothed rim and an inner diameter of a radial outwardly adjacent toothed-rim has a width between 0.2mm and 2.0mm, preferably 0.4mm and 1.2mm, more preferably between 0.8mm and 0.9mm.

**7.** The process according to any one of claims 4 to 6, wherein the first mixture (S1) dwells for between 0.01s and 0.004s, preferably between 0.02 and 0.07s, more preferably 0.01s +/-0.001s while passing the least two concentric toothed-rims (11, 12) of one of the rotor (10) and the stator and the at least one toothed-rim (21, 22) of the other of the rotor and the stator (20).

**8.** The process according to any one of claims 3 to 7, wherein edges (14, 24) of teeth (13, 23) along a flow path through the shearing geometry have rounded edges with a radius of at least 0.2 mm, in particular more than 3mm.

**9.** The process according to any one of claims 2 to 8, wherein the rotor (10) rotates at a speed relative to the stator between 250 and 7200, preferably between 1800 and 6000, more preferably between 4000 and 4500 revolutions per minute.

**10.** The process according to any one of claims 2 to 9, wherein the rotor (10) rotates at a peripheral speed between 2 m/s and 60 m/s, preferably between 15 m/s and 50 m/s, more preferably between 35 m/s and 45 m/s.

**11.** The process according to any one of claims 1 to 10, wherein the proportion of the volume of the first mixture (VM1, VM31) to the volume of the second tank (VK2, VK32) is in the range between ≥ 1% to ≤ 20%.

**12.** The process according to any one of claims 1 to 11, wherein the at least one beta-glucan is selected from the group consisting of *Schizophyllan* and *Scleroglucan,* wherein the *Schizophyllan* or *Scleroglucan* are obtained by fermentation of fungal strains.

**13.** The process according to any one of claims 1 to 12, wherein the fungal strains are *Schizophyllum commune* or *Sclerotium rolfsii*.

**14.** A process according to any one of claims 1 to 13, wherein the tank cascade further comprises a third tank (K33) with a third volume (VK33), and the process for fermentation further comprises at least the steps

d) transfer of the second mixture (S32) to the third tank (K33), and
e) fermentation of the fungal strains in the second mixture (S32) in a third aqueous medium (M33) in the third tank (K33),

wherein the proportion of the second mixture to the volume of the third tank (VK33) is in the range between ≥ 0.1 % to ≤ 50%.

**15.** The process according to claim 14, wherein the second mixture (S32) in step d) is passed through at least one high-shear mixer, the high-shear mixer (1) has a shearing geometry, such that the entire second mixture (S32) entirely passes through the shearing geometry of the at least one high-shear mixer.

**16.** The process according to any one of claims 14 and 15, wherein the proportion of the second mixture (S32) to the volume of the third tank (VK33) is in the range between ≥ 1 % to ≤ 20%.

**Patentansprüche**

**1.** Verfahren zur Vergärung von Pilzstämmen, die Glucane mit einer ß-1,3-glycosidisch verknüpften Hauptkette und Seitengruppen, die ß-1,6-glycosidisch daran gebunden sind, abscheiden, in einer Kaskade von Behältern, die mindestens einen ersten Behälter (K1, K31) mit einem ersten Volumen (VK1, VK31) und einen zweiten Behälter (K2, K32) mit einem zweiten Volumen (VK2, VK32) umfassen, das mindestens die folgenden Schritte umfasst

a) Vergärung der Pilzstämme in einem ersten wässrigen Medium (M1, M31) in dem ersten Behälter (K1, K31) und dem Volumen des ersten wässrigen Mediums (VM1, VM31), was zu einer ersten Mischung (S1, S31) führt,
b) Übertragen der ersten Mischung (S1, S31) zu dem zweiten Behälter (K2, K32) und
c) Gärung der Pilzstämme in der ersten Mischung (S1, S31) in einem zweiten wässrigen Medium (M2, M32) in dem zweiten Behälter (K2, K32) und dem Volumen des zweiten wässrigen Mediums (VM2, VM32), das zu einer zweiten Mischung (S2, S32) führt,

wobei das Verhältnis des Volumens der ersten Mischung (VM1, VM31) zu dem Volumen des zweiten Behälters (VK2, VK32) im Bereich zwischen ≥ 0,1 % bis ≤ 50 % liegt und wobei die erste Mischung (S1, S31) in Schritt b) durch mindestens einen Hochschermischer geführt wird, wobei der Hochschermischer (1) eine Schergeometrie aufweist, derart, dass die gesamte erste Mischung (S1, S31) vollständig durch die Schergeometrie des mindestens einen Hochschermischers hindurchgeht.

**2.** Verfahren nach Anspruch 1, wobei der Hochschermischer (1) ein Rotor-Stator-Mischer ist, der einen Rotor (10) und einen Stator (20) aufweist.

**3.** Verfahren nach Anspruch 2, wobei der Rotor-Stator-Mischer eine Dispergiermaschine mit gezahntem Rand ist.

**4.** Verfahren nach irgendeinem der Ansprüche 2 und 3, wobei mindestens einer von dem Rotor (10) und dem Stator des Rotor-Stator-Mischers mindestens zwei konzentrische gezahnte Ränder (11, 12) aufweist und der andere von dem Rotor und dem Stator (20) mindestens einen gezahnten Rand (21, 22) aufweist, wobei der mindestens eine gezahnte Rand des anderen von dem Rotor und dem Stator konzentrisch mit den mindestens zwei konzentrischen gezahnten Rändern verschachtelt ist, wobei das erste wässrige Medium (M1, M31) durch die verschachtelten gezahnten Ränder hindurchgeht.

**5.** Verfahren nach Anspruch 4, wobei die mindestens zwei konzentrisch gezahnten Ränder (11, 12) von einem von dem Rotor (10) und dem Stator und der mindestens eine gezahnte Rand (21, 22) des anderen von dem Rotor und dem Stator (20) eine abstandsgleiche Zahngeometrie aufweisen und wobei der Abstand zwischen nebeneinanderliegenden Zähnen (13) des jeweiligen äußeren gezahnten Rands (11) größer ist als der Abstand zwischen nebeneinanderliegenden Zähnen (23) des jeweiligen inneren gezahnten Rands (21), wobei das erste wässrige Medium M1 durch die verschachtelten gezahnten Ränder in einer Richtung von aufsteigendem Zahnabstand hindurchgeht.

**6.** Verfahren nach irgendeinem der Ansprüche 4 und 5, wobei die erste Mischung (S1) durch eine Lücke (2) in radialer Richtung hindurchgeht, welche Lücke in einer radialen Richtung durch die konzentrisch verschachtelten mindestens zwei konzentrisch gezahnten Rändern (11, 12) von einem von dem Rotor (10) und dem Stator und dem mindestens einen gezahnten Rand (21, 22) des anderen von dem Rotor und dem Stator (20) gebildet ist, wobei die Lücke (2)

zwischen einem Außendurchmesser eines gezahnten Rands und einem Innendurchmesser eines radial nach außen anliegenden gezahnten Rand eine Breite zwischen 0,2 mm und 2,0 mm, bevorzugt 0,4 mm und 1,2 mm, noch bevorzugter zwischen 0,8 mm und 0,9 mm aufweist.

**7.** Verfahren nach irgendeinem der Ansprüche 4 bis 6, wobei die erste Mischung (S1) sich 0,01 s bis 0,004 s, bevorzugt 0,02 bis 0,07 s, noch bevorzugter 0,01 s +/-0,001 s lang aufhält, während sie durch die mindestens zwei konzentrischen gezahnten Ränder (11, 12) von einem von dem Rotor (10) und dem Stator und dem mindestens einen gezahnten Rand (21, 22) des anderen von dem Rotor und dem Stator (20) hindurchgeht.

**8.** Verfahren nach irgendeinem der Ansprüche 3 bis 7, wobei Kanten (14, 24) von Zähnen (13, 23) einem Strömungsweg durch die Schergeometrie entlang abgerundete Kanten mit einem Radius von mindestens 0,2 mm, insbesondere mehr als 3 mm, aufweisen.

**9.** Verfahren nach irgendeinem der Ansprüche 2 bis 8, wobei der Rotor (10) sich mit einer Geschwindigkeit mit Bezug auf den Stator zwischen 250 und 7200, bevorzugt zwischen 1800 und 6000, noch bevorzugter zwischen 4000 und 4500 Umdrehungen pro Minute dreht.

**10.** Verfahren nach irgendeinem der Ansprüche 2 bis 9, wobei der Rotor (10) sich mit einer peripheren Geschwindigkeit zwischen 2 m/s und 60 m/s, bevorzugt zwischen 15 m/s und 50 m/s, noch bevorzugter zwischen 35 m/s und 45 m/s dreht.

**11.** Verfahren nach irgendeinem der Ansprüche 1 bis 10, wobei das Verhältnis des Volumens der ersten Mischung (VM1, VM31) zu dem Volumen des zweiten Behälters (VK2, VK32) im Bereich zwischen ≥ 1 % bis ≤ 20 % liegt.

**12.** Verfahren nach irgendeinem der Ansprüche 1 bis 11, wobei das mindestens eine Beta-Glucan aus der Gruppe ausgewählt ist bestehend aus Schizophyllan und Scleroglucan, wobei das Schizophyllan und Scleroglucan durch Gärung von Pilzstämmen erhalten werden.

**13.** Verfahren nach irgendeinem der Ansprüche 1 bis 12, wobei die Pilzstämme Schizophyllum commune oder Sclerotium rolfsii sind.

**14.** Verfahren nach irgendeinem der Ansprüche 1 bis 13, wobei die Behälterkaskade ferner einen dritten Behälter (K33) mit einem dritten Volumen (VK33) umfasst und das Gärungsverfahren ferner mindestens die Schritte umfasst

d) Überführen der zweiten Mischung (S32) zum dritten Behälter (K33) und
e) Gärung der Pilzstämme in der zweiten Mischung (S32) in einem dritten wässrigen Medium (M33) in dem dritten Behälter (K33),

wobei das Verhältnis der zweiten Mischung zu dem Volumen des dritten Behälters (VK33) im Bereich zwischen ≥ 0,1 % bis ≤ 50 % liegt.

**15.** Verfahren nach Anspruch 14, wobei die zweite Mischung (S32) in Schritt d) durch mindestens einen Hochschermischer hindurchgeht, wobei der Hochschermischer (1) eine Schergeometrie aufweist, derart, dass die gesamte zweite Mischung (S32) vollständig durch die Schergeometrie des mindestens einen Hochschermischers hindurchgeht.

**16.** Verfahren nach irgendeinem der Ansprüche 14 und 15, wobei das Verhältnis der zweiten Mischung (S32) zu dem Volumen des dritten Behälters (VK33) im Bereich zwischen ≥ 1 % bis ≤ 20 % liegt.

## Revendications

**1.** Procédé de fermentation de souches fongiques qui secrètent des glucanes ayant une chaîne principale à liaison β-1,3-glycosidique et des groupements latéraux y étant liés par voie β-1,6-glycosidique, dans des cuves en cascade comprenant au moins une première cuve (K1, K31) ayant un premier volume (VK1, VK31), et une deuxième cuve (K2, K32) ayant un deuxième volume (VK2, VK32), comprenant au moins les étapes

a) de fermentation des souches fongiques dans un premier milieu aqueux (M1, M31) dans la première cuve (K1, K31) et le volume du premier milieu aqueux (VM1, VM31), conduisant à un premier mélange (S1, S31),

b) de transfert du premier mélange (S1, S31) vers la deuxième cuve (K2, K32), et

c) de fermentation des souches fongiques dans le premier mélange (S1, S31) dans un deuxième milieu aqueux (M2, M32) dans la deuxième cuve (K2, K32) et le volume du deuxième milieu aqueux (VM2, VM32), conduisant à un deuxième mélange (S2, S32),

où la proportion du volume du premier mélange (VM1, VM31) par rapport au volume de la deuxième cuve (VK2, VK32) se trouve dans la plage allant de ≥ 0,1% à ≤ 50% et où on fait passer le premier mélange (S1, S31) dans l'étape b) à travers au moins un mélangeur à cisaillement élevé, le mélangeur à cisaillement élevé (1) possède une géométrie de cisaillement telle que l'ensemble du premier mélange (S1, S31) passe entièrement à travers la géométrie de cisaillement du au moins un mélangeur à cisaillement élevé.

**2.** Procédé selon la revendication 1, dans lequel le mélangeur à cisaillement élevé (1) est un mélangeur de type rotor-stator ayant un rotor (10) et un stator (20) .

**3.** Procédé selon la revendication 2, dans lequel le mélangeur de type rotor-stator est une machine de dispersion à couronnes dentées.

**4.** Procédé selon l'une quelconque des revendications 2 et 3, dans lequel au moins l'un parmi le rotor (10) et le stator du mélangeur de type rotor-stator possède au moins deux couronnes dentées concentriques (11, 12) et l'autre parmi le rotor et le stator (20) possède au moins une couronne dentée (21, 22), où la au moins une couronne dentée de l'autre parmi le rotor et le stator est entrelacée de manière concentrique avec les au moins deux couronnes dentées concentriques, où le premier milieu aqueux (M1, M31) passe à travers les couronnes dentées entrelacées.

**5.** Procédé selon la revendication 4, dans lequel les au moins deux couronnes dentées concentriques (11, 12) de l'un parmi le rotor (10) et le stator et la au moins une couronne dentée (21, 22) de l'autre parmi le rotor et le stator (20) possèdent une géométrie de dent équidistante et où la distance entre des dents adjacentes (13) de la couronne dentée externe respective (11) est plus grande que la distance entre des dents adjacentes (23) de la couronne dentée interne respective (21), où le premier milieu aqueux M1 passe à travers les couronnes dentées entrelacées dans le sens d'une distance de dents ascendante.

**6.** Procédé selon l'une quelconque des revendications 4 et 5, dans lequel le premier mélange (S1) passe à travers un espace (2) dans un sens radial, lequel espace dans un sens radial est formé par l'entrelacement de manière concentrique d'au moins deux couronnes dentées concentriques (11, 12) de l'un parmi le rotor (10) et le stator et la au moins une couronne dentée (21, 22) de l'autre parmi le rotor et le stator (20), où l'espace (2) entre un diamètre externe d'une couronne dentée et un diamètre interne d'une couronne dentée radiale extérieurement adjacente possède une largeur comprise entre 0,2 mm et 2,0 mm, préférablement entre 0,4 mm et 1,2 mm, plus préférablement entre 0,8 mm et 0,9 mm.

**7.** Procédé selon l'une quelconque des revendications 4 à 6, dans lequel le premier mélange (S1) séjourne pendant entre 0,01 s et 0,004 s, préférablement entre 0,02 et 0,07 s, plus préférablement 0,01 s ± 0,001 s lors de son passage via les au moins deux couronnes dentées concentriques (11, 12) de l'un parmi le rotor (10) et le stator et la au moins une couronne dentée (21, 22) de l'autre parmi le rotor et le stator (20).

**8.** Procédé selon l'une quelconque des revendications 3 à 7, dans lequel les bords (14, 24) des dents (13, 23) le long d'un chemin d'écoulement à travers la géométrie de cisaillement possèdent des bords arrondis ayant un rayon d'au moins 0,2 mm, en particulier supérieur à 3 mm.

**9.** Procédé selon l'une quelconque des revendications 2 à 8, dans lequel le rotor (10) tourne à une vitesse par rapport au stator comprise entre 250 et 7200, préférablement entre 1800 et 6000, plus préférablement entre 4000 et 4500 tours par minute.

**10.** Procédé selon l'une quelconque des revendications 2 à 9, dans lequel le rotor (10) tourne à une vitesse périphérique comprise entre 2 m/s et 60 m/s, préférablement entre 15 m/s et 50 m/s, plus préférablement entre 35 m/s et 45 m/s.

**11.** Procédé selon l'une quelconque des revendications 1 à 10, dans lequel la proportion du volume du premier mélange (VM1, VM31) par rapport au volume de la deuxième cuve (VK2, VK32) se trouve dans la plage allant de ≥ 1% à ≤ 20%.

**12.** Procédé selon l'une quelconque des revendications 1 à 11, dans lequel le au moins un bêta-glucane est choisi dans

le groupe constitué par le schizophyllane et le scléroglucane, où le schizophyllane et le scléroglucane sont obtenus par fermentation de souches fongiques.

**13.** Procédé selon l'une quelconque des revendications 1 à 12, dans lequel les souches fongiques sont *Schizophyllum commune* ou *Sclerotium rolfsii*.

**14.** Procédé selon l'une quelconque des revendications 1 à 13, dans lequel la cascade de cuves comprend en outre une troisième cuve (K33) ayant un troisième volume (VK33), et le procédé de fermentation comprend en outre au moins les étapes

d) de transfert du deuxième mélange (S32) vers la troisième cuve (K33), et
e) de fermentation des souches fongiques dans le deuxième mélange (S32) dans un troisième milieu aqueux (M33) dans la troisième cuve (K33),

où la proportion du deuxième mélange par rapport au volume de la troisième cuve (VK33) se trouve dans la plage allant de ≥ 0,1% à ≤ 50%.

**15.** Procédé selon la revendication 14, dans lequel on fait passer le deuxième mélange (S32) dans l'étape d) à travers au moins un mélangeur à cisaillement élevé, le mélangeur à cisaillement élevé (1) possède une géométrie de cisaillement telle que l'ensemble du deuxième mélange (S32) passe entièrement à travers la géométrie de cisaillement du au moins un mélangeur à cisaillement élevé.

**16.** Procédé selon l'une quelconque des revendications 14 et 15, dans lequel la proportion du deuxième mélange (S32) par rapport au volume de la troisième cuve (VK33) se trouve dans la plage allant de ≥ 1% à ≤ 20%.

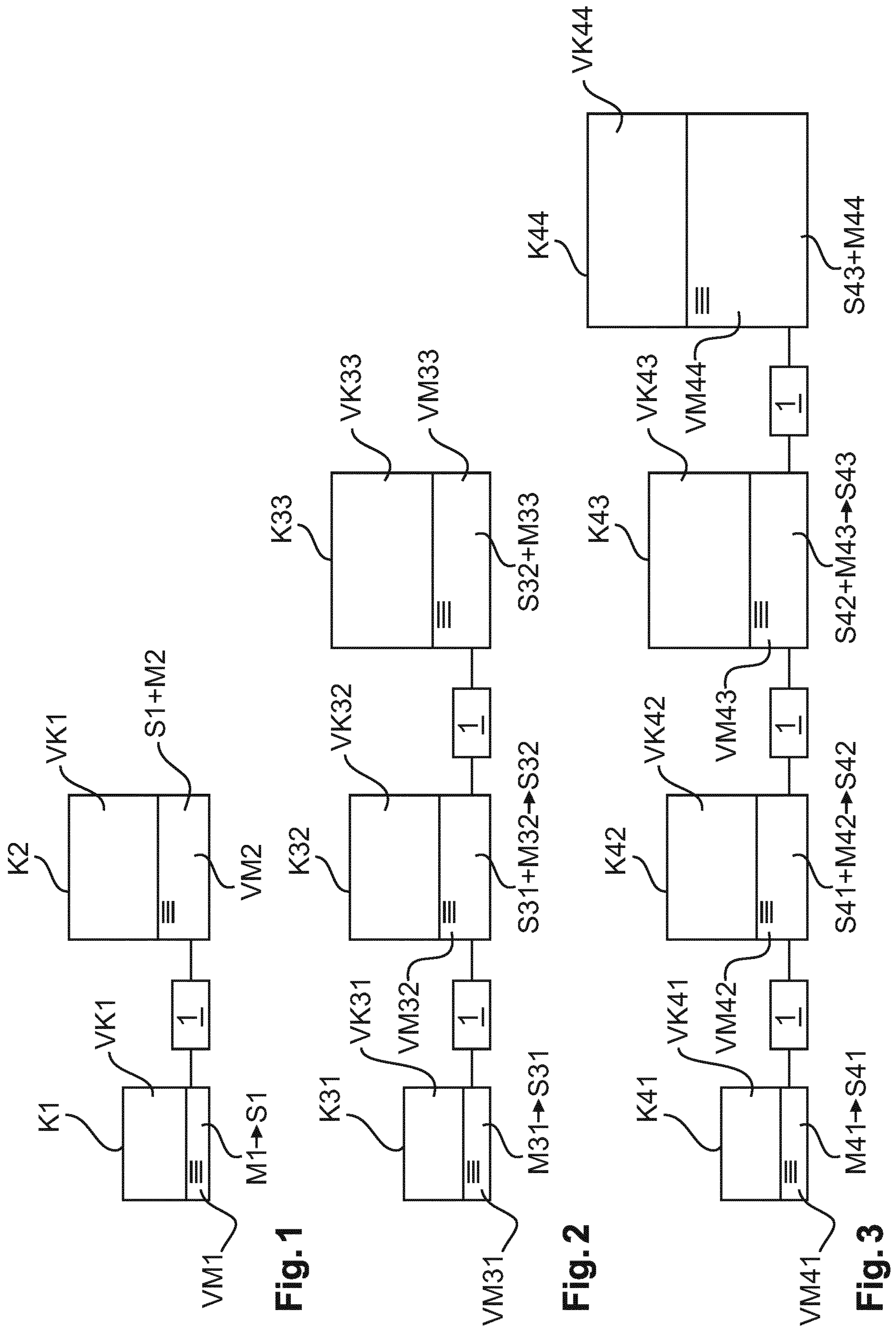


Fig. 1

Fig. 2

Fig. 3

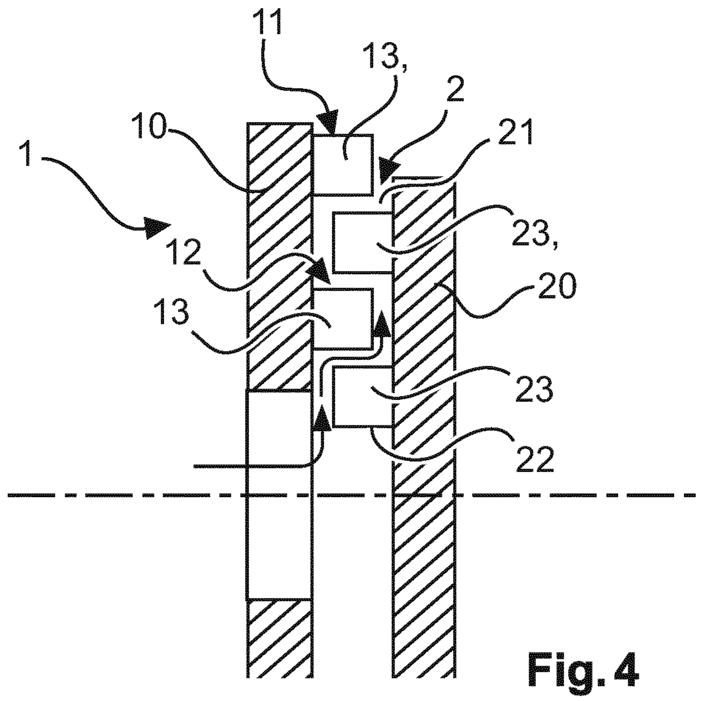


Fig. 4

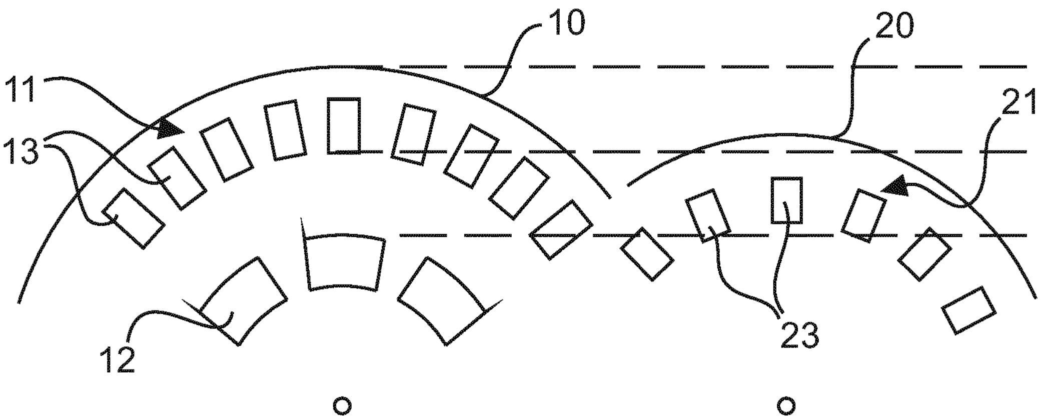


Fig. 5a

Fig. 5b

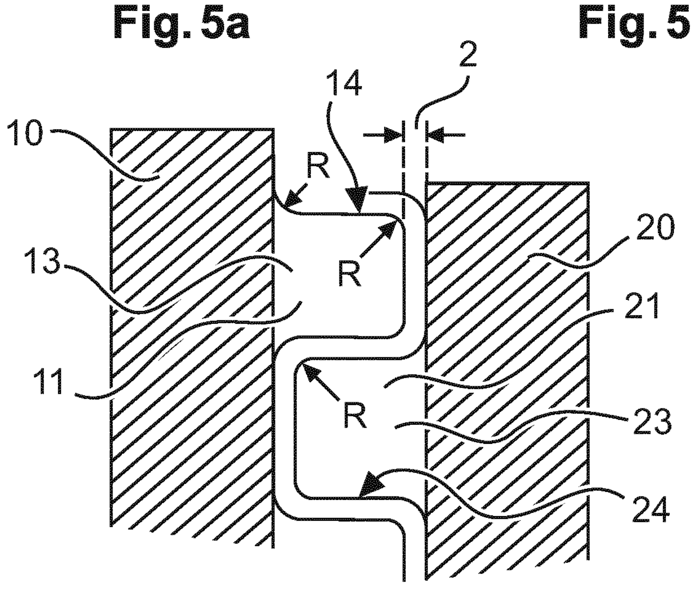


Fig. 6

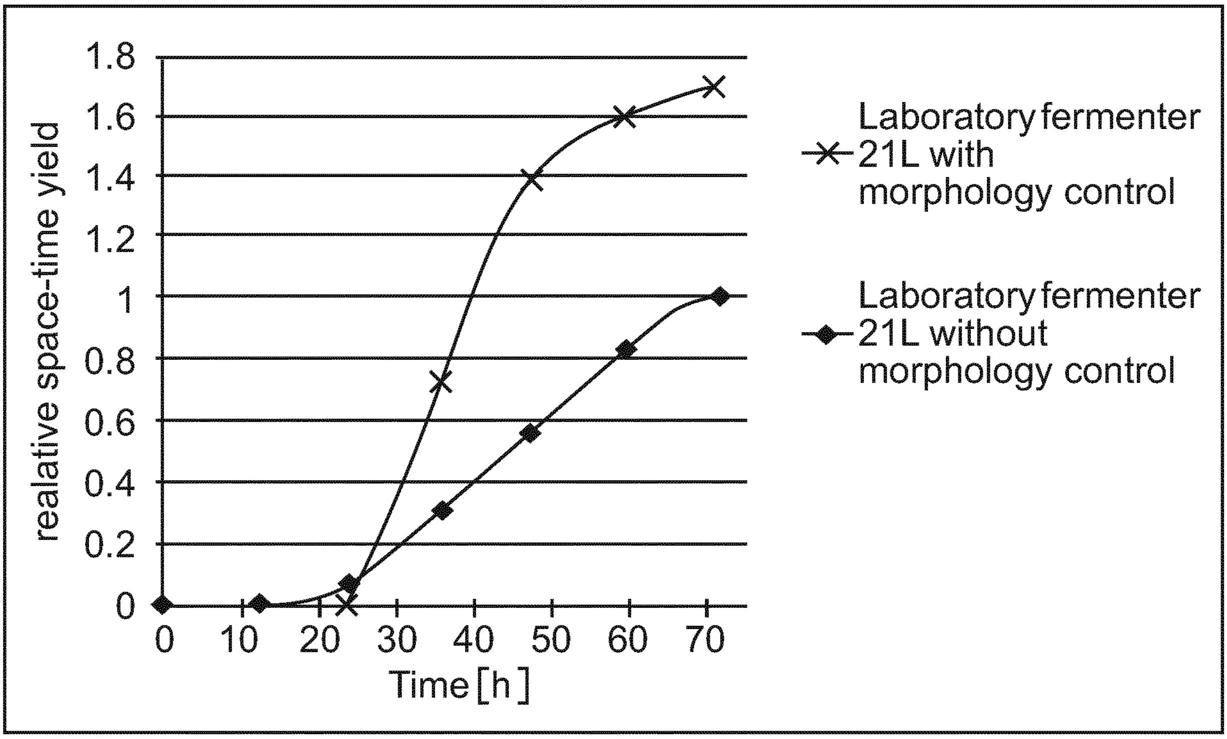
1.8
1.6
1.4
1.2
1
0.8
0.6
0.4
0.2
0
realative space-time yield
0
10
20
30
40
50
60
70
Time [h]
Laboratory fermenter 21L with morphology control
Laboratory fermenter 21L without morphology control

**Fig. 7**

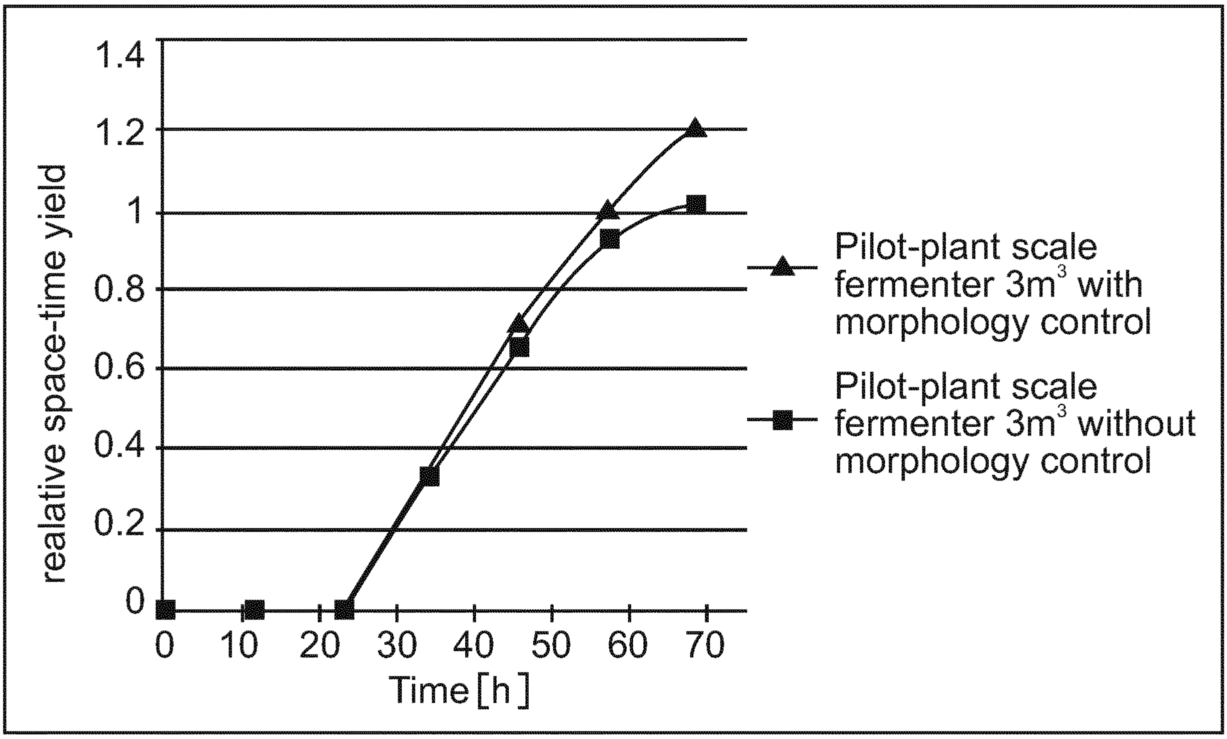
1.4
1.2
1
0.8
0.6
0.4
0.2
0
realative space-time yield
0
10
20
30
40
50
60
70
Time [h]
Pilot-plant scale fermenter 3m³ with morphology control
Pilot-plant scale fermenter 3m³ without morphology control

**Fig. 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 463540 B1
- EP 0271907 A2
- EP 0504673 A1
- DE 4012238 A1
- WO 03016545 A2
- US 5010186 A
- US 4873323 A
- US 4667026 A
- EP 0504673 A
- EP 5046073 A
- EP 0271907 A


### Non-patent literature cited in the description


- **NOVAK ; ENDOCRINE.** *Metabol & Immune Disorders - Drug Targets,* 2009, vol. 9, 67-75
- **STAHMANN.** *Appl Environ Microbiol,* 1992, vol. 58, 3347-3354
- **KIM.** *Biotechnol Letters,* 2006, vol. 28, 439-446
- **NIKITINA.** *Food Technol Biotechnol,* 2007, vol. 45, 230-237
- **SURVASE.** *Food Technol Biotechnol,* 2007, 107-118
- Biosynthese, Produktion und Eigenschaften von extrazellulären Pilz-Glucanen. **UDO RAU.** Habilitationsschrift. Technical University of Brunswick, 1997, 70-95
- **UDO RAU.** Biopolymers. WILEY-VCH Publishers, 2002, vol. 6, 63-79
- *GIT Fachzeitung Labor,* vol. 12/92, 1233-1238
- **BRIGAND.** Industrial Gums. Academic Press, 1993, 461-472
- Biosynthese, Produktion und Eigenschaften von extrazellulären Pilz- Glucanen [Biosynthesis, production and properties of extracellular fungal glucans. **UDO RAU.** Postdoctoral thesis. Technical University of Braunschweig, 1997
- **MELIN ; RAUTENBACH.** Membranverfahren. Springer-Verlag, 2007, 309-366
- **MELIN ; RAUTENBACH.** Membranverfahren. Springer-Verlag,, 2007, 51-52
- **MELIN ; RAUTENBACH.** Membranverfahren. Springer-Verlag, 2007, 20
- **U. RAU ; R. -J. MÜLLER ; K. CORDES ; J. KLEIN.** Process and molecular data of branched 1,3-β-D-glucans in comparison with Xanthan. *Bioprocess Engineering,* 1990, vol. 5 (2), 89-93
- Biosynthese, Produktion und Eigenschaften von extrazellulären Pilz-Glucanen [Biosynthesis, production and properties of extracellular fungal glucans. **UDO RAU.** Postdoctoral thesis. Technical University of Braunschweig, 1997
- **U. RAU ; C. BRANDT.** Oxygen controlled batch cultivations of Schizophyllum commune for enhanced production of branched β-1,3-glucans. *Bioprocess Engineering,* September 1994, vol. 11 (4), 161-165